(19) Europäisches Patentamt
European Patent Office
Office européen des brevets

(11) **EP 2 454 990 A2**

(12) **EUROPÄISCHE PATENTANMELDUNG**

(43) Veröffentlichungstag:
**23.05.2012 Patentblatt 2012/21**

(51) Int Cl.:
**A61B 5/053** (2006.01)

(21) Anmeldenummer: **11009056.0**

(22) Anmeldetag: **15.11.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **17.11.2010 DE 102010051459**

(71) Anmelder: **Pötzschke, Harald**
**65191 Wiesbaden (DE)**

(72) Erfinder: **Pötzschke, Harald**
**65191 Wiesbaden (DE)**

(74) Vertreter: **Müller, Claudia**
**European Patent Attorney**
**Uhlandstrasse 58**
**60314 Frankfurt / Main (DE)**

(54) **Wundüberwachung mit textilen Meßgrößenwandler-Systemen**

(57) Die vorliegende Erfindung betrifft ein Verfahren zur Überwachung des Zustandes von Wunden durch Ermittlung spezifischer Kenngrößen, insbesondere der Temperatur, der Feuchte, und/oder der Volumenzunahme des verwundeten Gewebes, mittels flexibler, weicher textiler Messgrößenwandler-Systeme, mit einem oder mehreren Messgrößenwandlern aus elektrisch leitenden Metallen in Form von Fasern oder Garnen, oder als Beschichtung von Fasern oder Garnen, die aus einem elektrisch nicht-leitenden Material bestehen. Dabei werden die genannten spezifischen Kenngrößen durch Messung elektrischer Widerstände (ohmscher Widerstand (R), kapazitiver Widerstand ($X_c = (\omega C)^{-1}$), induktiver Widerstand ($X_L = \omega L$)) ermittelt. Die Messgrößenwandler-Systeme können sowohl unter oder zwischen Wundauflagen gelegt, als auch strukturell in Wundauflagen integriert werden. Wird mit ihrer Hilfe der Zustand von Wunden überwacht, ist ein Verbandswechsel nur zur Ermittlung des Zustandes von Wunden nicht erforderlich.

**Fig. 1**

EP 2 454 990 A2

## Beschreibung

### Gegenstand der Erfindung

Die vorliegende Erfindung betrifft ein Verfahren zur Überwachung des Zustandes von Wunden durch Ermittlung spezifischer Kenngrößen, insbesondere der Temperatur, der Feuchte, und/oder der Volumenzunahme des verwundeten Gewebes, mittels flexibler, weicher textiler Messgrößenwandler-Systeme, mit einem oder mehreren Messgrößenwandlern aus elektrisch leitenden Metallen in Form von Fasern oder Garnen, oder als Beschichtung von Fasern oder Garnen, die aus einem elektrisch nicht-leitenden Material bestehen. Dabei werden die genannten spezifischen Kenngrößen durch Messung elektrischer Widerstände (ohmscher Widerstand (R), kapazitiver Widerstand ($X_c = (\omega C)^{-1}$), induktiver Widerstand ($X_L = \omega L$)) ermittelt. Die Messgrößenwandler-Systeme können sowohl unter oder zwischen Wundauflagen gelegt, als auch strukturell in Wundauflagen integriert werden. Wird mit ihrer Hilfe der Zustand von Wunden überwacht, ist ein Verbandswechsel nur zur Ermittlung des Zustandes von Wunden nicht erforderlich.

### Hintergrund der Erfindung

#### Wundheilung

Die regelhafte Heilung chirurgisch versorgter Hautwunden kann durch Komplikationen gestört oder unterbrochen werden.

Die häufigsten Komplikationen sind bakterielle Infektionen, Nahtrisse mit Dehiszenzen der Wundränder und Blutungen, sowie die Ausbildung von Hämatomen oder Seromen im Gewebe unter der chirurgischen Naht.

#### Überwachung der Wundheilung

Die Überwachung des Zustandes von Wunden sowie der Fortentwicklung der Wund heilung geschieht üblicherweise durch einen planmäßigen Verbandwechsel mit Inspektion der Wunde (ev. mit Palpation der Wundumgebung) durch einen Therapeuten (ärztliche oder Pflege-Person).

Jeder Verbandwechsel aber ist ein Risiko, denn er gibt Keimen die Möglichkeit zum Zutritt zur Wunde.

Die Regeln der medizinischen Hygiene verbieten eine Wiederverwendung der gebrauchten Wundauflage.

Die Möglichkeit des Verzichtes auf einen Verbandwechsel nur zum Zwecke der Inspektion der Wunde, sowie des Belassens eines liegenden, nicht zu beanstandenden Wundverbandes - weil der Zustand der Wunde und möglicherweise auch der Fortgang der Wundheilung aus den Messwerten von Sensoren kontinuierlich ermittelt wird - verspricht ökonomische Ressourcen (Arbeitszeiten und Materialien) zu sparen. Eine mit einer solchen kontinuierlichen Überwachung des Zustandes einer Wunde möglicherweise einhergehende frühzeitige Erfassung von Komplikationen der Wundheilung ist zudem förderlich für den Heilungsverlauf.

Die häufigsten Komplikationen chirurgisch versorgter Wunden können durch eine Ermittlung von drei Zustandsbeschreibenden Größen der Wunde oder der unmittelbaren Wundumgebung erfasst werden.

1) Der Verlauf der Temperatur der Wund-nahen Hautoberfläche kann bakterielle Entzündungen anzeigen.

2) Der Verlauf der Feuchte kann eine Blutung oder Sekretion nach einem Nahtriss, sowie eine Sekretion aufgrund einer bakteriellen Infektion anzeigen.

3) Bei fixierten (bspw. angeklebten) Rändern der Wundauflage kann eine Zunahme der Dehnung der Wundauflage die Entwicklung einer Raumforderung unter der Naht, also bspw. die Entwicklung eines größeren Hämatoms oder Seroms, anzeigen.

### Stand der Technik

Kommerziell erhältliche Messgrößenwandler oder Wundauflagen mit einem integrierten System zur messtechnischen Überwachung des Zustandes einer Wunden mit den geforderten Eigenschaften konnten nicht ermittelt werden.

Den Stand der Technik beschreibt folgende Patentliteratur:

Die Patentschrift US 5,181,905 (FLAM: "Method of Monitoring the Condition of the Skin or Wound") beschreibt Wundauflagen mit flachen Indikator-Behältem die auf der Oberseite der Wundauflagen befestigt, oder von oben in die Wundauflage eingesenkt sind, wobei die Wundauflage "Informationen" an Indikatoren, die sich in dem Indikatorhalter befinden, weiterleitet und die Indikatoren ohne Verbandwechsel von außen abgelesen werden können: In der Wundauflage bis in den Indikatorhalter vorgedrungenes Wundsekret erlaubt die Ermittlung der Azidität (pH) und des Feuchtegrades (Sekretmenge), physikalische Informationen wie die Temperatur oder der Druck werden stofflos auf Sensoren im Indikatorgefäß weitergeleitet.

Diese Vorrichtung erlaubt keine direkte Erfassung von Messwerten auf der Oberfläche einer Hautwunde, die Messungen erfolgen ausschließlich in fortgeleitetem Wundsekret, und eine kontinuierliche Messung ist nicht möglich.

Die Patentanmeldung US 2010/0268111 (DRINAN et al.: "Monitoring Platform for Wound and Ulcer Monitoring and Detection ") beschreibt eine Wundauflage zur therapeutischen lektrostimulation und zur Messung der Impedanz in der Wunde und deren Umgebung. Die Wundauflage enthält eine Anzahl eingelagerter flächiger Elektroden, die Messung der elektrischen Impedanz zwischen jeweils zwei dieser Elektroden erlaubt die Bestimmung des Wassergehaltes (der Hydratation) des Gewebes zwischen diesen Elektroden.

Diese Wundauflagen erlauben nur die Ermittlung des Wassergehaltes im Gewebe nahe einer Wunde, aber keine direkte Erfassung von Messwerten auf der Oberfläche einer Haut wunde, außerdem sind die Elektroden hart und können Druckstellen hervorrufen. Außerdem besitzt die Vorrichtung keine offene Struktur, die ein Ableiten von Flüssigkeiten in eine Wundauflage ermöglicht.

Die Patentanmeldungen EP 2 127 694 A1 (KONINKLIJKE PHILIPS ELECTRONICS N:V:: "Multi-Electrode Path for Monitoring and Electrical Stimulation of Wound Healing") und WO 2009/144615 A1 (KONINKLIJKE PHILIPS ELECTRONICS N:V:: "Moisture Control Within a Multi-Electrode Patch for Monitoring and Electrical Stimulation of Wound Healing") beschreiben eine Vorrichtung zur therapeutischen Elektrostimulation von Wunden. Die Vorrichtung besteht aus einer weichen "Substanz" (eine Trägerfolie / Auflage) mit vielen eingelagerten flächigen Elektroden (in Form einer zweidimensionalen Matrix), die beim Auflegen auf eine Wunde Kontakt mit dieser und der peri-ulceralen Haut erhalten. Durch Vermessen der elektrischen Widerstände zwischen jeweils 2 Elektroden kann auch eine Topik der Feuchteverteilung unter dem beweglichen Elektrodenträger ermittelt werden. Diese Vorrichtung kann nur verwendet werden, wenn eine Wundauflage entfernt wurde, und nur zur direkten Messung der Feuchte. Das Prinzip erscheint für eine längere Anwendung unter einer Wundauflage nicht geeignet, da die Vorrichtung viele flächige Elektroden enthält, die selbst durch Druck, etc. die Wundheilung negativ beeinflussen können. Außerdem besitzt die Vorrichtung keine offene Struktur, die ein Ableiten von Flüssigkeiten in eine Wundauflage ermöglicht.

Die Patentanmeldung US 2008/0103550 A1 (WENZEL et al.: "Multiple Electrode Wound Healing Patch") beschreibt therapeutische Auflagen mit Elektroden zur Elektrostimulation von Wunden. Diese Vorrichtung kann nur verwendet werden, wenn ein Wundverband entfernt wurde, und erhebt keinen Anspruch auf Messfunktionen. Außerdem besitzt die Vorrichtung keine offene Struktur, die ein Ableiten von Flüssigkeiten in eine Wundauflage ermöglicht.

Patentliteratur zu Wundauflagen mit integrierten Systemen zur Überwachung des Zustandes von Wunden mit den geforderten Eigenschaften konnte keine ermittelt werden.

Wie ersichtlich, sind in der Patentliteratur bisher noch keine vergleichbaren Messsysteme zur Wundüberwachung bzw. Wundauflagen mit einem integrierten System zur messtechnischen Überwachung und nachfolgender datenmäßiger Ermittlung des Zustandes einer Wunden mit den geforderten Eigenschaften beschrieben, wobei der Verband / die Wundauflage nicht entfernt werden muss.

## Aufgabe der Erfindung

Der Erfindung liegt die Aufgabe zugrunde, ein geeignetes Verfahren und Vorrichtungen (Messgrößenwandler-Systeme) für eine kontinuierliche Überwachung von Wunden, insbesondere bezüglich einer frühzeitigen Erkennung von Komplikationen der Wundheilung, durch die messtechnische Erfassung geeigneter Kenngrößen (Messgrößen) der Wunde und der unmittelbaren Wundumgebung zu entwickeln, ohne das ein Wundverband entfernt werden muss. Die Vorrichtungen sollen unter einer Wundauflage liegen, oder, als ein besonderer Aspekt der Aufgabe, auf möglichst viele bekannte Wundauflagen einfach aufgebracht bzw. darinnen integriert werden können.

Die Vorrichtungen sollen hinreichend weich und flexibel sein, damit sie keinen Druck auf die Wunde oder die Haut in der Wundumgebung ausüben, sowie möglichst flach und mit offener Struktur sein, damit Blut oder Wundsekret ohne Behinderung hindurch gelangen und von einer saugfähigen Wundauflage aufgenommen werden können.

Es sollen mehrere Messgrößen gleichzeitig und kontinuierlich erfasst werden können.

## Lösung der Aufgabe / Kurzbeschreibung der Erfindung

Die Erfindung löst die geschilderte Aufgabe durch ein Verfahren zur insbesondere auch kontinuierlichen Wundüberwachung mit erfindungsgemäß bereitgestellten flexiblen, weichen, textilen Messgrößenwandler-Systemen (mit einem oder mehreren Messgrößenwandlern). Diese Messgrößenwandler-Systeme können sowohl unter oder zwischen Wundauflagen gelegt, als auch strukturell in Wundauflagen integriert werden. Somit betrifft die Erfindung ein Verfahren zur Überwachung von Wunden, das dadurch gekennzeichnet ist, dass Kenngrößen der Wunde und der unmittelbaren Wundumgebung mittels textiler Messgrößenwandler-Systeme erfasst und in elektrische Signale gewandelt, verarbeitet und angezeigt werden.

Vorzugsweise sind die textilen Messgrößenwandler-Systeme unter einer Wundauflage auf einer Wunde platziert, oder kommen strukturell auf oder in Wundauflagen integriert zum Einsatz.

Ganz besonders bevorzugt sind die Messgrößenwandler-Systeme zur Überwachung von Wunden Textilien, sie bestehen also aus einem Gewebe, einem Geflecht, einem Gewirk, einem Gestrick, oder einem Vlies.

Erfindungsgemäße Messgrößenwandler-Systeme enthalten ein oder mehrere elektrische Messgrößenwandler, die aus elektrisch leitenden Fasern, oder aus Garnen mit einem Anteil aus elektrisch leitenden Fasern, die entweder elektrisch isoliert oder elektrisch nicht-isoliert sind, bestehen.

Derartige Messgrößenwandler-Systeme sind insbesondere dadurch gekennzeichnet, dass die elektrisch leitenden Fasern, oder die Garne mit einem Anteil aus elektrisch leitenden Fasern, ihren resistiven, kapazitiven oder induktiven Widerstand in Folge der Wirkung der zu messenden Größe ändern.

Erfindungsgemäße Meßgrößenwandler-Systeme können bevorzugt strukturell auf eine

Wundauflage aufgebracht werden, oder in eine nicht-textile Wundauflage integriert werden können, indem sie bei deren Herstellung als Textilie in die Masse der nicht-textilen Wundauflage eingearbeitet werden.

Bevorzugt sind erfindungsgemäße Messgrößenwandler-Systeme der oben beschriebenen Art, welche Textilien sind, die aus einer textilen Wundauflage und den Messgrößenwandlem bestehen.

Derartige Messgrößenwandler-Systeme können zur Überwachung von Wunden verwendet werden, wobei sie als Teil eines Sensorsystems zur Wundüberwachung auf eine Wunde unter einer Wundauflage platziert werden, oder auf die Oberfläche nicht-textiler Wundauflagen aufgebracht, oder in deren Substanz oberflächlich eingebettet oder in die Tiefe eingelassen sind, oder ihre textile Struktur eine textile Wundauflage oder ein Teil einer textilen Wundauflage ist.

Insbesondere werden erfindungsgemäß spezifische Kenngrößen der Wunde und der unmittelbaren Wundumgebung, ausgewählt aus Temperatur, Feuchte, Volumenzustand, oder Kombinationen hiervon, mittels textiler Messgrößenwandler-Systeme durch Ermittlung eines oder mehrerer Widerstandswerte, ausgewählt aus ohmschem, kapazitivem und induktivem Widerstand, erfasst und in elektrische Signale gewandelt, verarbeitet und angezeigt. Bevorzugt werden die Feuchte und die Temperatur in Kombination, -oder die Feuchte und die Temperatur in Kombination mit dem Volumenzustand gemessen. Erfindungsgemäß umfassen Messgrößenwandler-Systeme zur kontinuierlichen Überwachung von Wunden Textilien, ausgewählt aus einem Gewebe, einem Geflecht, einem Gewirk, einem Gestrick, oder einem Vlies, umfassend einen oder mehrere elektrisch leitende Meßgrößenwandler.

Die textilen Messgrößenwandler bestehen aus elektrisch leitenden Metallen in der Form (1) von (nicht-beschichteten) Fasern oder Garnen, oder als Beschichtung von Fasern oder Garnen, die aus einem elektrisch nicht-leitenden Material bestehen (sie bilden dann die Hülle einer mehrschichtigen Faser oder eines mehrschichtigen Games), oder (2) von elektrisch isolierend beschichteten Fasern oder Garnen (sie bilden darin den Kern einer mehrschichtigen Faser oder eines mehrschichtigen Games).

Die genannten elektrisch leitenden Fasern oder Game sind vorzugsweise zusammen mit elektrisch nicht-leitenden Fasern oder Garnen zu flexiblem Textilien (Geweben, Geflechten, Gewirken, Gestricken oder Vliesen) verarbeitet und die Leiterfasem oder -game, je nach Messgröße, entsprechend planvoll elektrisch-leitend mit einander verbunden.

Die primäre Messgröße der textilen Messgrößenwandler ist die Änderung eines elektrischen Widerstandes, und zwar eines resistiven, eines kapazitiven oder eines induktiven Widerstandes, oder eine Kombination solcher Änderungen, wobei eine bekannte funktionale Abhängigkeit der Änderungen von der Bestimmungsgröße existiert.

Die Erfassung der elektrischen Widerstandsänderungen erfolgt mit einer Signalverarbeitungseinheit, die die Signale verarbeitet sowie anzeigt.

**Ausführliche Beschreibung der Erfindung**

Bedeutung in dieser Schrift verwendeter Begriffe

| | |
|---|---|
| Faser | Ein im Verhältnis zur Länge sehr dünnes und flexibles Gebilde, Grundbestandteil aller Textilien. |
| Garn | Fadenförmiger Verbund von Fasern; eine Textilie kann technisch aus Einzelfasern, oder aus Garnen hergestellt werden. |
| Textilie | Ein flexibles Material, das aus einem Verbund von Fasern besteht (insbesondere Gewebe, Geflechte, Gewerke, Gestricke oder Vliese). |
| elektrische leitende Fasern (und Game) | werden hier auch allgemein Leiter, Leiterfasem, oder Messgrößenwandlerfasern, etc. genannt, übliche elektro-technische Bezeichnungen sind Drähte (einadrig) und Drahtbündel (mehradrig), Leiterfolien sind flache Drähte, Leiterschläuche sind bspw. in einer Richtung aufgedrehte Folien oder eine Faser oder einem Garn umhüllende Leiter, als Kabel werden üblicherweise elektrisch isolierte Leiter (also Verbünde mit anderen Materialien) bezeich- |

net.

| Textiles Messgrößenwandler-System | Ein Textil mit integrierten Messgrößenwandlem, die durch ein textiltechnologisches Verfahren aus textilen Fasern hergestellt werden (also insbesondere Gewebe, Geflechte, Gewerke, Gestricke oder Vliese). |
| Textile Wundauflagen | Wundauflagen, die durch ein textiltechnologisches Verfahren aus textilen Fasern hergestellt werden (also insbesondere Gewebe, Geflechte, Gewerke, Gestricke oder Vliese). Bekannte Beispiele sind Gazen oder Kompressen, bspw. aus Baumwolle (z. B. Mullkompressen). |
| Nicht-textile Wundauflagen | Wundauflagen, deren zur Wunde weisende Oberfläche aus einer amorphen Substanz besteht, bspw. Kunststoffschäume, Gele, Kolloide, Lyofilisate, etc. |

Messgrößenwandler-System

Die erfindungsgemäßen textilen Messgrößenwandler werden als System in ein Trägertextil strukturell integriert.

Mit den erfindungsgemäßen textilen Messgrößenwandler-Systemen werden elektrische Widerstände und daraus Kenngrößen des Wundzustandes (Temperatur, Feuchte, Volumenzunahme des verwundeten Gewebes) bestimmt.

Die Widerstände sind ohmsche (R), kapazitive ($X_c = (\omega C)^{-1}$) oder induktive Widerstände ($X_L = \omega L$).

i) Temperatur

In einem elektrisch isolierten Leiter ändert sich der spezifische Widerstand in bekannter Weise temperaturabhängig und somit der Gesamtwiderstand zwischen den beiden Drahtenden.

Eine besonders geeignete Anordnung für einen textilen Messgrößenwandler kann vorzugsweise ein Mäander oder eine Spirale eines elektrisch isolierten Leiter sein.

ii) Feuchte

Für textile Messgrößenwandler-Systeme zur Ermittlung der Feuchte sind mehrere Messverfahren möglich.

1) Messung eines resistiven Widerstandes:

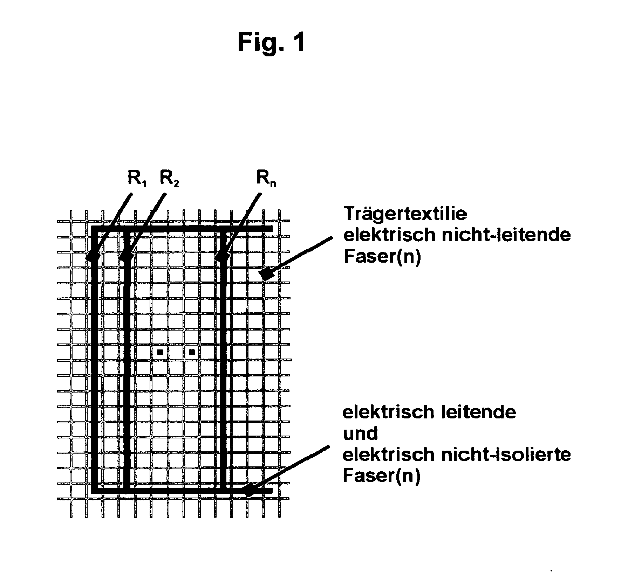

Im Verlauf eines nicht-isolierten Leiters kann es durch Wundsekret oder sonstige Feuchte zu einen Stromfluss durch diese Flüssigkeit kommen, was zu einer Reduktion des messbaren Gesamtwiderstandes des Leiters führt. Es können auch mehrere solcher Leiter parallel geschaltet werden, indem die gleichseitigen Enden aller Leiterfasem elektrisch miteinander verbunden werden (siehe Figur 1), wodurch eine größere Fläche sensorisch erfasst werden kann. Weitere geeignete Anordnungen elektrisch nicht-isolierter Leiter sind Mäander oder Spiralen.

2) Messung eines kapazitiven Widerstands:

Zwischen zwei parallel verlaufenden isolierten Leitern kann sich durch Wundsekret oder sonstige Feuchte das Dielektrikum - und damit die relative Permittivität $\varepsilon_R$ - zwischen den Leitern und somit auch der kapazitive Widerstand ($X_c \sim \varepsilon_R$) zwischen jeweils einem Ende der beiden parallelen Leiter ändern.

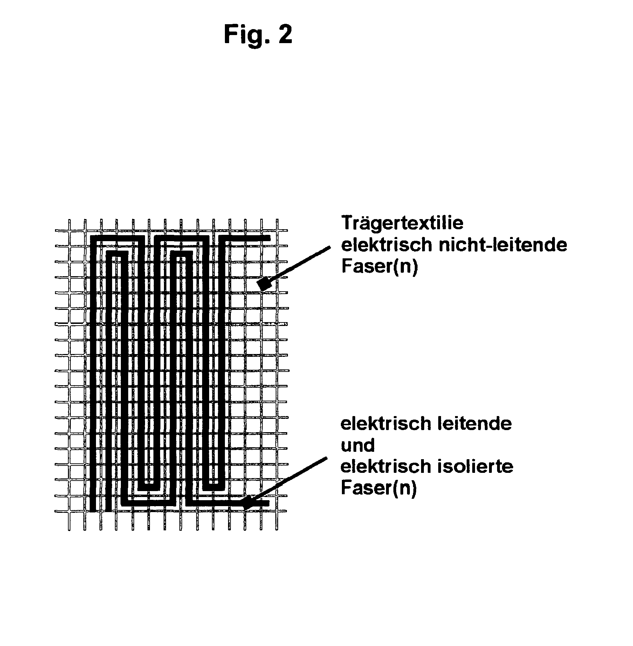

Um eine integrative Erfassung einer größeren Fläche der Wundauflage zu ermöglichen sind bevorzugte Anordnungen ein mäandrierender oder ein spiraliger Verlauf von zwei parallelen Leitern (siehe Figur 2).

iii) Dehnung als Maß für eine Volumenzunahme des Gewebes

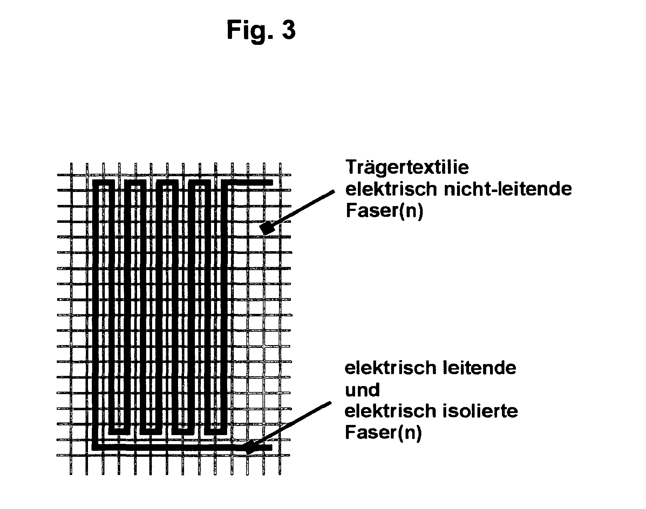

Eine funktionale elektrische Spule in einem textilen Messgrößenwandler-System, bestehend aus einem isolierten Leiter mit einem mäanderförmigen Verlauf (siehe Figur 3), erfährt bei Dehnung eine Veränderung ihrer Induktivität L und damit eine Änderung des induktiven Widerstandes ($X_L$) zwischen den beiden Leiterenden.

Um Dehnungen in den beiden Dimensionen der Ebene detektieren zu können, muss der Mäanders vorzugsweise in dem textilen Messgrößenwandler-System in zwei Ausrichtungen angeordnet werden, oder zwei unabhängige Messgrößenwandler-System vorhanden sein.

Eine weitere vorteilhafte Anordnung zur Erzeugung eines spulenähnlichen Gebildes ist eine Spirale einer elektrisch isolierten Leiterfaser.

Signalverarbeitung

Die physikalischen Größen (ohmsche Widerstand R, Kapazität C und Induktivität L) der Messgrößenwandler-Systeme können mittels einfacher elektronischer Schaltungen gleichzeitig und kontinuierlich erfasst werden.

Dazu werden elektrische Einheiten zur Spannungs- und / oder Stromversorgung der Messgrößenwandler, sowie zur Erfassung (Sensorelektronik), und gegebenenfalls zur Anzeige, sonstigen Kommunikation mit dem Therapeuten oder Patienten (Warnsignalgeber, etc.) oder Speicherung der Mess- und/oder Bestimmungswerte (Datenspeicher), an die textilen Messgrößenwandler-Systeme angeschlossen. Zur Verbindung mit den elektrischen Einheiten werden die Messgrößenwandler-Systeme mit bekannten elektrischen Kontakten versehen.

Benutzung der textilen Messgrößenwandler-Systeme mit Wundauflagen

Die erfindungsgemäßen textilen Messgrößenwandler-Systeme können sehr einfach auf eine Wunde gelegt und mit Wundauflagen bedeckt, oder auch zwischen Wundauflagen gelegt werden.

Integration der textilen Messgrößenwandler-Systeme auf oder in Wundauflagen

Die erfindungsgemäßen Messgrößenwandler können auch sehr einfach in andere Textilien, bspw. textile Wundauflagen, integriert werden, indem die Fasern oder Garne der Messgrößenwandler im Verlauf der Herstellung der textilen Wundauflage anstelle anderer Fasern oder Garne, oder zusätzlich, in die textile Wundauflage eingearbeitet (eingewebt, eingeflochten, eingewirkt, eingestrickt, etc.) werden.

Dies ermöglicht, sie direkt in textile Wundauflagen einzuarbeiten.

Die Messgrößenwandler können auch auf die wundseitige Oberfläche textiler Wundauflagen beispielsweise genäht oder gestickt werden.

Die Messgrößenwandler-Systeme können auch in nicht-textile Wundauflagen, wie Lyofilisate oder erhärtende Gießmassen, im Verlauf von deren Herstellung eingebettet oder eingebracht und so strukturell integriert werden.

Sie können auch auf die Oberfläche nicht-textiler Wundauflagen bspw. geklebt werden

**Ausgestaltungsbeispiele**

Anhand der beigefügten Zeichnungen werden exemplarisch Ausführungsformen der erfindungsgemäßen textilen Messgrößenwandler-Systeme, mit denen das erfindungsgemäße Verfahren zur Wundüberwachung durchgeführt werden kann, näher beschrieben.

Figur 1     Schematische Darstellung einer Anordnung der Leiter eines Messgrößenwandlers zur Feuchtebestimmung durch Messung eines resistiven Widerstandes.

Figur 2     Schematische Darstellung einer Anordnung der Leiter eines Messgrößenwandlers zur Feuchtebestimmung durch Messung eines kapazitiven Widerstandes

Figur 3     Schematische Darstellung einer Anordnung der Leiter eines Messgrößenwandlers zur Dehnungserfassung durch Messung eines induktiven Widerstandes.

Die Figuren zeigen im Einzelnen:

Figur 1

zeigt beispielhaft eine mögliche technische Ausgestaltung eines Messgrößenwandlers für die Bestimmung der Feuchte durch Messung eines resistiven Widerstandes.

Werden nicht-isolierte faser- oder garnförmige Leiter ohne sich zu berühren (bspw. abwechselnd mit elektrisch nicht-leitenden Fasern als Abstand erhaltende Strukturen) parallel in einem Textil angeordnet und die Enden aller Leiter des Messgrößenwandlers auf den beiden Seite des Textils jeweils leitend miteinander verbunden, ergibt sich zwischen diesen Seiten ein so genanntes Widerstandsnetzwerk von n (= Anzahl der Sensorfasern) parallel angeordneten ohmschen Widerständen ($R_1$ bis $R_n$). Jeder einzelne Draht besitzt dabei einen ohmschen Widerstand $R_i$. Für den gesamten ohmschen Widerstand $R_{FS}$ gilt:

$$R_{FS}^{-1} = \Sigma\ R_i^{-1}\ (i = 1, 2, \ldots, n)$$

Kommt es zu einer Benetzung des Widerstandsnetzwerkes mit einer den elektrischen Strom leitenden Flüssigkeit, insbesondere Wundsekret, Blut oder Eiter, führt dies zu einem elektrischen Kurzschluss zwischen Fasern des Messgrößenwandlers mit einer Änderung des Gesamtwiderstandes $R_{FS}$ des Netzwerkes. Diese Änderung ist vom Ausmaß und der räumlichen Ausprägung der Benetzung abhängig.

Eine weitere mögliche technische Ausgestaltung eines Messgrößenwandlers für die Bestimmung der Feuchte durch Messung eines resistiven Widerstandes ist eine mäandrierende Führung (ähnlich wie in der Figur 3 gezeigt) nur eines elektrisch nicht-isolierten Leiters.

Figur 2

zeigt beispielhaft ein Schema einer möglichen technischen Ausgestaltung eines Messgrößenwandlers für die Bestimmung der Feuchte durch Messung eines kapazitiven Widerstandes. Verlaufen elektrisch isolierte Leiter in einem etwa gleichen Abstand parallel und mäanderförmig in einer Textilie, ist diese Struktur ein zweidimensionales (flächiges) kondensatorähnliches Gebilde, das eine Kapazität C (und damit einen kapazitiven Widerstand $X_c$) besitzt. Ist der Raum zwischen den beiden Leitern mit einer textilen Struktur gefüllt, besteht das Dielektrikum mit der relativen Permittivität $\varepsilon_R$ des erzeugten kondensatorähnlichen Gebilde im Wesentlichen aus Luft (und Textilie). Kommt es zu einer Benetzung der textilen Struktur mit einer Flüssigkeit, führt dies zu einer Veränderung des Dielektrikums und damit zu einer Veränderung des kapazitiven Widerstandes.

Figur 3

zeigt beispielhaft (in Form einer schematischen Darstellung) eine mögliche technische Ausgestaltung der Anordnung der Leiter eines Messgrößenwandlers zur Dehnungserfassung durch Messung eines induktiven Widerstandes. Wird ein elektrisch isolierter Leiter mäanderförmig in einer textilen Struktur angeordnet, kann ein zweidimensionales (flächiges) spulenähnliches Gebilde hergestellt werden, das eine Induktivität L (und damit einen induktiven Widerstand $X_L$) besitzt. Die Induktivität hängt dabei von der Geometrie (Anzahl der Mäanderschleifen und deren Abstand voneinander) ab, sie bleibt für eine gewählte Geometrie konstant. Ändert sich die Geometrie beispielsweise durch Dehnung der textilen Struktur, ändert sich die Abstände zwischen den Schleifen und damit die Induktivität.

**Patentansprüche**

1.  Verfahren zur Überwachung von Wunden,
    **dadurch gekennzeichnet,**
    **dass** Kenngrößen der Wunde und der unmittelbaren Wundumgebung mittels textiler Messgrößenwandler-Systeme erfasst und in elektrische Signale gewandelt, verarbeitet und angezeigt werden.

2.  Verfahren zur kontinuierlichen Überwachung von Wunden gemäß Anspruch 1,
    **dadurch gekennzeichnet, dass** spezifische Kenngrößen der Wunde und der unmittelbaren Wundumgebung, ausgewählt aus Temperatur, Feuchte, Volumenzustand oder Kombinationen hiervon, mittels textiler Messgrößenwandler-Systeme durch Ermittlung eines oder mehrerer Widerstandswerte, ausgewählt aus ohmschem, kapazitivem und induktivem Widerstand, erfasst und in elektrische Signale gewandelt, verarbeitet und angezeigt werden.

3.  Verfahren zur Überwachung von Wunden nach Anspruch 1 oder 2, **dadurch gekennzeichnet, dass** die textilen Messgrößenwandler-Systeme unter einer Wundauflage auf einer Wunde platziert werden, oder strukturell auf oder in Wundauflagen integriert zum Einsatz kommen.

4.  Messgrößenwandler-Systeme zur kontinuierlichen Überwachung von Wunden,
    **dadurch gekennzeichnet, dass** die Messgrößenwandler-Systeme aus Textilien, ausgewählt aus einem Gewebe, einem Geflecht, einem Gewirk, einem Gestrick, oder einem Vlies, umfassend einen oder mehrere elektrisch leitende Messgrößenwandler, bestehen.

5.  Messgrößenwandler-Systeme gemäß Anspruch 4,
    **dadurch gekennzeichnet, dass** sie als elektrisch leitende Anteile ein oder mehrere Messgrößenwandler enthalten, die aus elektrisch leitenden Fasern, oder aus Garnen mit einem Anteil aus elektrisch leitenden Fasern bestehen, die entweder elektrisch isoliert oder elektrisch nicht-isoliert sind.

6.  Messgrößenwandler-Systeme gemäß Anspruch 4 oder Anspruch 5,

**dadurch gekennzeichnet, dass** sie elektrisch leitende Fasern, oder Game mit einem Anteil aus elektrisch leitenden Fasern zur Erfassung der Änderung ihres resistiven, und/oder kapazitiven, und/oder induktiven Widerstands in Folge der Wirkung der zu messenden Kenngröße aufweisen.

7. Messgrößenwandler-Systeme gemäß einem der Ansprüche 4, 5 oder 6,
   **dadurch gekennzeichnet, dass** sie strukturell in eine Wundauflage integriert sind.

8. Messgrößenwandler-Systeme gemäß einem der Ansprüche 4, 5 oder 6,
   **dadurch gekennzeichnet, dass** sie strukturell in eine nicht-textile Wundauflage integriert sind.

9. Textile Wundauflagen-Kombination, bestehend aus einem Messgrößenwandler-System gemäß einem der Ansprüche 4, 5 oder 6, und einer textilen Wundauflage.

10. Verwendung von Messgrößenwandler-Systemen gemäß einem der Ansprüche 4, 5 oder 6 zur Überwachung von Wunden,
    auf einer Wunde unter einer Wundauflage, oder
    auf der Oberfläche nicht-textiler Wundauflagen, oder
    in der Wundauflagen-Substanz oberflächlich oder in der Tiefe eingebettet, oder
    als textile Struktur einer textilen Wundauflage, oder
    als ein Teil einer textilen Wundauflage.

# Fig. 1

R₁ R₂ Rₙ

Trägertextilie
elektrisch nicht-leitende
Faser(n)

elektrisch leitende
und
elektrisch nicht-isolierte
Faser(n)

# Fig. 2

Trägertextilie
elektrisch nicht-leitende
Faser(n)

elektrisch leitende
und
elektrisch isolierte
Faser(n)

# Fig. 3

Trägertextilie
elektrisch nicht-leitende
Faser(n)

elektrisch leitende
und
elektrisch isolierte
Faser(n)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5181905 A
- US 20100268111 A
- EP 2127694 A1
- WO 2009144615 A1
- US 20080103550 A1